# EUROPEAN PATENT SPECIFICATION

(11) **EP 4 231 971 B1**
(45) Date of publication and mention of the grant of the patent: **27.11.2024**
(21) Application number: 21802000.6
(22) Date of filing: 20.10.2021
(51) Int. Cl.: A61F 2/44, A61N 5/06

(54) **ORTHOPEDIC PROSTHESIS, PARTICULARLY INTERSOMATIC CAGE**
ORTHOPÄDISCHE PROTHESE, INSBESONDERE INTERSOMATISCHER KÄFIG
PROTHÈSE ORTHOPÉDIQUE, EN PARTICULIER CAGE INTERSOMATIQUE

(30) Priority: 23.10.2020 IT 202000025192
(43) Date of publication of application: 30.08.2023
(73) Proprietor: SPS S.r.l., 10010 Scarmagno (TO) (IT)
(72) Inventor: VALLANA, Valerio, 10123 Torino (IT)
(74) Representative: Buzzi, Notaro & Antonielli d'Oulx S.p.A.
(86) International application number: PCT/IB2021/059658
(87) International publication number: WO 2022/084877

(56) References cited:
- WO-A1-96/39932
- FR-A1- 2 929 504
- US-A1- 2005 175 658
- US-A1- 2010 317 948
- US-A1- 2012 109 304
- US-A1- 2015 282 941
- US-B1- 7 815 682

## Description

### Field of the invention

The present invention generally relates to the orthopaedic prosthesis such as, by way of non-limiting example, the intersomatic cages for vertebral stabilisation designed to be inserted between two contiguous vertebrae so as to space them apart and therefore keep them at a mutual distance such to restore the intervertebral space, creating the decompression of the nerve roots and the acceleration of the intersomatic fusion.

### State of the art

Intersomatic cages thus made typically comprise a body provided with external anchoring formations and with a nose protruding from the front end of the body and designed to be inserted between two contiguous vertebrae during the surgical insertion of the intersomatic cage.

Following implantation of the cage, the patient should normally be treated from a pharmaceutical point of view in order to avoid the onset of infections as much as possible. Typically, treatment consists in the administration of antibiotics, which however reveal two types of problems: the risk of the patient developing a resistance which reduces or even nullifies antibiotic efficacy, and the onset of possible adverse reactions that can develop even more serious infections.

Similar problems occur with orthopaedic prostheses in general.

It is therefore desirable to avoid the use of antibiotics in postoperative hospitalisation, replacing them with safer and at the same time not less effective alternative measures.

Documents US 2012/109304, US 2005/175658 and WO 2006/041670 disclose orthopaedic prostheses incorporating leds with controlled emission of ultraviolet rays. The efficacy of UV radiations in terms of sterilising, disinfecting and antibacterial properties is per se known: for example, the United States Patent application US 2006/0183987 describes a venous implant for cancer phototherapy consisting of a ring of UV rays emitter leds which can be connected to an electrical power source by means of a cable.

### Summary of the invention

The object of the present invention is to improve the functional efficacy of an intersomatic cage provided with leds with controlled emission of ultraviolet rays and such object is achieved thanks to the characteristics set out in the claims below.

Thanks to the invention, the intersomatic cage allows to provide, following the implantation thereof, a more effective selective treatment of the orthopaedic region and of the vertebral region in this case. In particular, the miniaturised leds are distributed superficially on the faces of the cage, specifically applied to the anchoring indentations, and they are connected to an activation circuit, also miniaturised and integrated in the cage, power-supplied by an external and remote electrical source. The power supply is conveniently wireless, carried out for example by induction or by means of radio frequency (RF) power waves.

### Brief description of the drawings

The invention will now be described in detail with reference to the attached drawings, provided purely by way of non-limiting example, wherein:

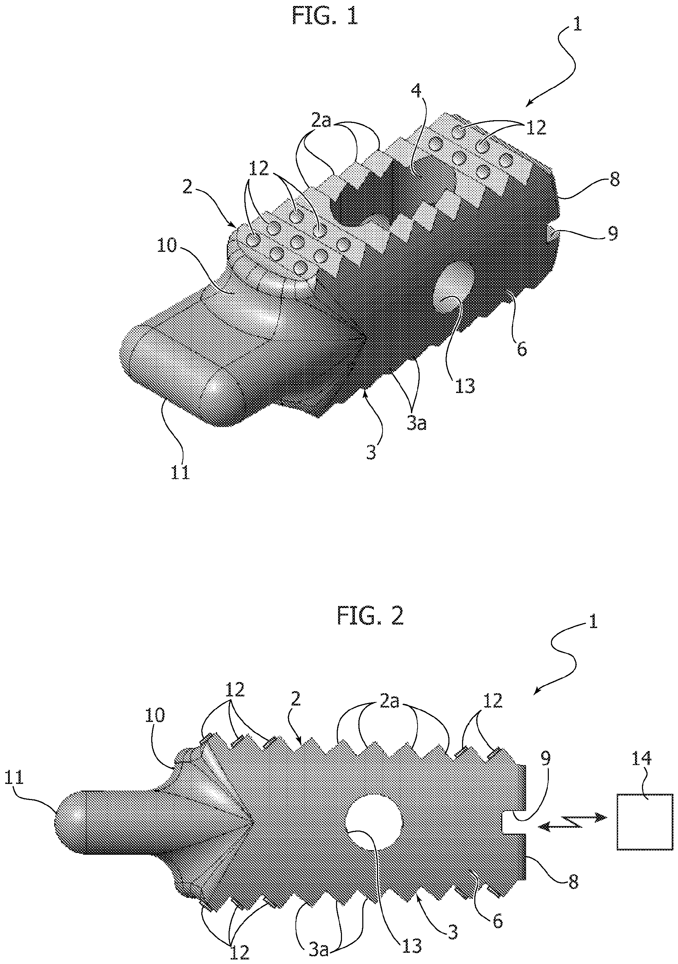

- figure 1 is a schematic perspective view of an orthopaedic prosthesis according to the invention,
- figure 2 is a diagram exemplifying the power supply of the prosthesis according to the invention.

### Detailed description of the invention

The embodiment described herein refers to the particular case of an intersomatic cage for vertebral stabilisation. It should be observed that the invention is generally equally advantageously applicable to any orthopaedic prosthesis.

With reference to the figures, the intersomatic cage according to the invention consists of a generally prismatic-shaped and more precisely parallelepiped-shaped monolithic body 1, having an upper face 2 and a lower face 3 (with reference to the implanted position of the cage in the intervertebral space of a subject with an upright vertebral column), both formed with respective anchoring formations consisting of parallel indentations 2a, 3a.

A slot-shaped through opening 4 vertically traverses the body 1 between the upper 2 and lower 3 faces, and a circular through hole 5 extends between the lateral walls 6, 7 of the body 1.

As observable in figure 4, the body 1 has a height, measured between the upper 2 and lower 3 faces, which decreases slightly toward the rear end thereof, indicated with 8 and in which a recess 9 for the introduction of an instrument for the surgical insertion of the cage into the intervertebral space. This instrument, not illustrated, is configured so as to engage axially and torsionally with the recess 9 so as to be able to rotate the body 1 alternately in a clockwise and anti-clockwise direction while being simultaneously pushed.

A nose 11 - which has the function of paving the way by dilating the intervertebral space during the process for inserting the intersomatic cage - protrudes from the front end of the body 1, indicated with reference numeral 10. The nose 11 extends parallel to the upper 2 and lower 3 faces of the body 1 and it has a substantially quadrangular shape with a constant cross-section and rounded edges. More precisely, the nose 11 extends on the extension of a median longitudinal plane of the body 1 and therefore protrudes from a median area of the front end 10, connecting to such median area through curved surfaces, preferably having variable radiuses, clearly visible in the figures.

According to the distinctive characteristic of the invention, the body 1 of the cage incorporates a plurality of leds 12 with controlled emission of ultraviolet rays.

The miniaturised leds 12 are distributed superficially at least on the faces 2, 3 of the cage and more particularly they are applied on at least part of the anchoring formations consisting of the parallel indentations 2a, 3a.

As clearly observable in the drawings, the leds 12 are advantageously fixed on the front inclined walls (with reference to the direction of insertion of the cage in the intervertebral space) of at least part of the indentations 2a, 2b. In this manner, in the implanted condition of the cage, the leds will be directly in contact with the vertebral regions.

The leds 12 are operatively connected to an activation or energisation circuit 13, which is also miniaturised and preferably, though not necessarily, integrated in the cage body 1. The activation circuit 13 is power-supplied by an internal or external electrical source: in the latter case, power is conveniently transmitted in wireless mode and is carried out for example by induction or by means of a remote emitter 14 of radio frequency (RF) power waves.

Alternatively, the activation circuit 13 may be provided for outside the cage, and the electrical power source thereof could also consist of a battery housed in the body 1 or arranged externally.

The leds 12 with controlled emission of ultraviolet rays may also be provided for on other parts of the body 1, for example also on the nose 11.

Obviously, the construction details and the embodiments may widely vary with respect to what has been described and illustrated, without departing from the scope of protection of the invention as described in the claims that follow.

## Claims

1. Orthopaedic prosthesis, in particular intersomatic cage for vertebral stabilisation, comprising a generally parallelepiped body (1) having two respectively upper and lower opposite faces (2, 3), with reference to an implanted position of the cage in the intervertebral space of a subject with an upright vertebral column, provided with respective anchoring formations (2a, 3a), said prosthesis incorporating a plurality of leds (12) with controlled emission of ultraviolet rays, **characterised in that** the leds (12) with controlled emission of ultraviolet rays are arranged at least on the upper and lower faces (2, 3) of the body (1) of the cage and they are applied on at least part of said anchoring formations (2a, 3a).

2. Orthopaedic prosthesis according to claim 1, **characterised in that** the leds (12) with controlled emission of ultraviolet rays are applied on the front surfaces, with reference to the direction of insertion of the cage in the intervertebral space, of said anchoring formations (2a, 3a).

3. Orthopaedic prosthesis according to claim 1 or 2, **characterised in that** the leds (12) with controlled emission of ultraviolet rays are operatively connected to an activation circuit (13).

4. Orthopaedic prosthesis according to claim 3, **characterised in that** the activation circuit (13) is integrated in the body (1) of the prosthesis.

5. Orthopaedic prosthesis according to claim 3 or 4, **characterised in that** the activation circuit (13) is supplied by a remote electrical source (14).

6. Orthopaedic prosthesis according to any one of claims 3 a 5, **characterised in that** the activation circuit (13) is power-supplied in wireless mode.

7. Orthopaedic prosthesis according to claim 3 or 4, **characterised in that** the activation circuit (13) is power-supplied by a battery.

8. Orthopaedic prosthesis according to claim 7, **characterised in that** the intersomatic cage comprises a nose (11) protruding from the front end (10) of the body (1) and designed to be inserted between two contiguous vertebrae to space them apart them during the surgical insertion of the cage, the nose (11) extending parallel to said upper and lower faces (2, 3) and being substantially quadrangular-shaped with constant cross-section and rounded edges, the leds (12) with controlled emission of ultraviolet rays being also arranged on said nose (11).

## Patentansprüche

1. Orthopädische Prothese, insbesondere intersomatischer Käfig zur vertebralen Stabilisierung, umfassend einen im Allgemeinen quaderförmigen Körper (1) mit zwei entsprechend oberen und unteren gegenüberliegenden Seiten (2, 3), mit Bezug auf eine implantierte Position des Käfigs im Intervertebralraum eines Subjekts mit einer aufrechten Wirbelsäule, versehen mit entsprechenden Verankerungsformationen (2a, 3a), wobei die Prothese eine Vielzahl von LEDs (12) mit kontrollierter Emission von Ultraviolettstrahlung enthält, **dadurch gekennzeichnet, dass** die LEDs (12) mit kontrollierter Emission von Ultraviolettstrahlung mindestens auf der oberen und der unteren Seite (2, 3) des Körpers (1) des Käfigs angeordnet sind und auf mindestens einen Teil der Verankerungsformationen (2a, 3a) angewendet werden.

2. Orthopädische Prothese nach Anspruch 1, **dadurch gekennzeichnet, dass** die LEDs (12) mit kontrollierter Emission von Ultraviolettstrahlung auf die Vorderseiten, mit Bezug auf die Richtung des Einsetzens des Käfigs in den Intervertebralraum, der Verankerungsformationen (2a, 3a) angewendet wird.

3. Orthopädische Prothese nach Anspruch 1 oder 2, **dadurch gekennzeichnet, dass** die LEDs (12) mit kontrollierter Emission von Ultraviolettstrahlung operativ mit einer Aktivierungsschaltung (13) verbunden sind.

4. Orthopädische Prothese nach Anspruch 3,
**dadurch gekennzeichnet, dass** die Aktivierungsschaltung (13) in den Körper (1) der Prothese integriert ist.

5. Orthopädische Prothese nach Anspruch 3 oder 4, **dadurch gekennzeichnet, dass** die Aktivierungsschaltung (13) von einer entfernten Stromquelle (14) versorgt wird.

6. Orthopädische Prothese nach einem der Ansprüche 3 bis 5, **dadurch gekennzeichnet, dass** die Aktivierungsschaltung (13) im drahtlosen Modus mit Strom versorgt wird.

7. Orthopädische Prothese nach Anspruch 3 oder 4, **dadurch gekennzeichnet, dass** die Aktivierungsschaltung (13) von einem Akku mit Strom versorgt wird.

8. Orthopädische Prothese nach Anspruch 7, **dadurch gekennzeichnet, dass** der intersomatische Käfig eine Nase (11) umfasst, die von dem vorderen Ende (10) des Körpers (1) vorspringt und dazu gestaltet ist, zwischen zwei aufeinanderfolgende Wirbel eingesetzt zu werden, um sie während des chirurgischen Einsetzens des Käfigs auseinander zu halten, wobei sich die Nase (11) parallel zu der oberen und der unteren Seite (2, 3) erstreckt und im Wesentlichen viereckig mit konstantem Querschnitt und abgerundeten Ecken ist, wobei die LEDs (12) mit kontrollierter Emission von Ultraviolettstrahlung ebenfalls an der Nase (11) angeordnet sind.

## Revendications

1. Prothèse orthopédique, en particulier cage intersomatique pour stabilisation vertébrale, comprenant un corps généralement parallélépipédique (1) ayant deux faces supérieure et inférieure (2, 3) respectivement opposées, en référence à une position implantée de la cage dans l'espace intervertébral d'un sujet à colonne vertébrale droite, pourvues de formations d'ancrage (2a, 3a) respectives, ladite prothèse incorporant une pluralité de DEL (12) à émission contrôlée de rayons ultraviolets, **caractérisée en ce que** les DEL (12) à émission contrôlée de rayons ultraviolets sont agencées au moins sur les faces supérieure et inférieure (2, 3) du corps (1) de la cage et qu'elles sont appliquées sur au moins une partie desdites formations d'ancrage (2a, 3a) .

2. Prothèse orthopédique selon la revendication 1, **caractérisée en ce que** les DEL (12) à émission contrôlée de rayons ultraviolets sont appliquées sur les faces avant, en référence au sens d'insertion de la cage dans l'espace intervertébral, desdites formations d'ancrage (2a, 3a).

3. Prothèse orthopédique selon la revendication 1 ou 2, **caractérisée en ce que** les DEL (12) à émission contrôlée de rayons ultraviolets sont connectées fonctionnellement à un circuit d'activation (13).

4. Prothèse orthopédique selon la revendication 3, **caractérisée en ce que** le circuit d'activation (13) est intégré dans le corps (1) de la prothèse.

5. Prothèse orthopédique selon la revendication 3 ou 4, **caractérisée en ce que** le circuit d'activation (13) est alimenté par une source électrique distante (14).

6. Prothèse orthopédique selon l'une quelconque des revendications 3 à 5, **caractérisée en ce que** le circuit d'activation (13) est alimenté en électricité dans un mode sans fil.

7. Prothèse orthopédique selon la revendication 3 ou 4, **caractérisée en ce que** le circuit d'activation (13) est alimenté en électricité par une batterie.

8. Prothèse orthopédique selon la revendication 7, **caractérisée en ce que** la cage intersomatique comprend un nez (11) faisant saillie à partir de l'extrémité avant (10) du corps (1) et conçu pour être inséré entre deux vertèbres contiguës pour les écarter lors de l'insertion chirurgicale de la cage, le nez (11) s'étendant parallèlement auxdites faces supérieure et inférieure (2, 3) et étant de forme sensiblement quadrangulaire avec une section constante et des bords arrondis, les DEL (12) à émission contrôlée de rayons ultraviolets étant également agencées sur ledit nez (11).
